# EUROPÄISCHE PATENTSCHRIFT

(11) **EP 4 279 033 B1**
(45) Veröffentlichungstag und Bekanntmachung des Hinweises auf die Patenterteilung: **24.06.2026**
(21) Anmeldenummer: 23201401.9
(22) Anmeldetag: 24.02.2021
(51) Int. Cl.: A61B 17/72, A61F 2/28

(54) **EXTRAKORPORAL LÄNGENVERSTELLBARES IMPLANTATSYSTEM SOWIE LÄNGENVERSTELLBARE IMPLANTATKOMPONENTE**
EXTRACORPOREAL DRIVE UNIT FOR AN EXTRACORPOREAL LENGTH-ADJUSTABLE IMPLANT SYSTEM, AND LENGTH-ADJUSTABLE IMPLANT SYSTEM
UNITÉ D'ENTRAÎNEMENT EXTRACORPORELLE POUR UN SYSTÈME D'IMPLANT À LONGUEUR EXTRA-CORPOREL RÉGLABLE ET SYSTÈME D'IMPLANT À LONGUEUR RÉGLABLE

(30) Priorität: 25.02.2020 EP 20159426
(43) Veröffentlichungstag der Anmeldung: 22.11.2023
(62) Teilanmeldung aus: 21706310.6
(73) Patentinhaber: Waldemar Link GmbH & Co. KG, 22339 Hamburg (DE)
(72) Erfinder: FISCHER, Hans-Joachim, 22850 Norderstedt (DE); LINK, Helmut D., 22397 Hamburg (DE); DMUSCHEWSKY, Klaus, 22339 Hamburg (DE)
(74) Vertreter: Alatis

(56) Entgegenhaltungen:
- EP-A2- 1 611 868
- CN-A- 102 895 048
- US-A1- 2004 030 395
- US-A1- 2011 230 883
- US-A1- 2017 049 489
- US-B1- 7 753 915

## Beschreibung

Die Erfindung betrifft ein von außen (extrakorporal) verstellbares System zur Längenverstellung einer Implantatkomponente als Teil einer Endoprothese sowie eine Verstelleinrichtung dafür. Die Verstelleinrichtung ist vorgesehen für einen Prothesenschaft der Implantatkomponente, die zur Befestigung an einem zu verlängernden Knochen ausgebildet ist, und umfasst gegeneinander verschiebliche Befestigungsteile, von denen jedes an einem Teil des zu verlängernden Knochens anzuordnen ist, sowie ein von einem Antriebselement angetriebenes Verstellelement, das dazu ausgebildet ist die zwei gegeneinander verschieblichen Befestigungsteile zu distrahieren.

Intrakorporal verstellbare Prothesen haben Bedeutung für An wendungsfälle, bei denen Prothesen sozusagen mitwachsen sollen. Sie sind insbesondere in solchen Fällen indiziert, wo entweder aufgrund natürlicher Vorgänge, wie beispielsweise beim Wachstum von Kindern, eine entsprechend längere Prothese benötigt wird, aber auch für solche Anwendungsfälle, bei denen zur Knochenbildung (Osteogenese) der Knochen entlang einer Trenn- bzw. Bruchstelle zusehends weiter gestreckt wird, um so fortlaufend die Bildung neuer Zellen anzuregen. Beispielsweise werden bei letzterem Anwendungsfall die zwei durch die Trenn stelle entstanden Knochenteile sukzessive voneinander wegbewegt, beispielsweise um 1 mm pro Tag, um so ständig die Bildung von neuem Knochengewebe anzuregen. Auf diese Weise kann beispielsweise eine Extremität, wie ein Bein, verlängert werden. Das ist von Bedeutung, um einen Längenausgleich zwischen den beiden Beinen eines Menschen herzustellen. In ähnlicher, wenn auch in der Regel deutlich langsamerer Weise, kann auch durch beständiges Erhöhen der Länge einer Prothese erreicht werden, dass der mit der Prothese versorgte Knochen in einer ähnlichen Weise länger wird, wie es beim natürlichen Wachstumsprozess von Kindern oder Jugendlichen der Fall wäre.

Bereits seit langem ist bekannt, dass für solche Fälle externe Fixateure verwendet werden. Diese weisen jedoch gravierende Nachteile auf, beispielsweise sind sie unhandlich sowie unfallgefährlich, und ferner haben sie ein hohes Risiko für Infektionen.

Um diesen Nachteilen zu begegnen, sind implantierbare Prothesen entwickelt worden, die eine gewünschte Längenverstellung mittels einer intrakorporal implantierten Prothese ermöglichen. Die eigentliche Längenverstellung kann hierbei invasiv erfolgen, insbesondere bei einer rein mechanischen Längenverstellung, oder mittels einem an der Prothese implantierten An trieb, der von außen ferngesteuert wird (Mutars-System der deutschen Firma Implantcast GmbH, Synoste-System der finnischen Firma Synoste Oy).

Letztere bieten den Vorteil, dass die Verstellung non-invasiv von außendurchgeführt werden kann. Dazu enthält die Prothese einen Elektromotor bzw. bildet einen Teil eines Elektromotors, der von außen (extrakorporal) angesteuert werden kann. Ggf. kann er auch von außen mit Energie versorgt werden. So ist ein solcher Aufbau mit einem sozusagen verteilten Elektromotor bekannt, bei dem die Prothese und ihr Verstellelement ein Teil eines Elektromotors bildet (sozusagen den Rotor), und der andere Teil mit der Erregung (sozusagen der Stator) von einem extrakorporale Aufsatz gebildet ist (WO 01/786141). Der eigentliche Elektromotor ist also im Grunde teilweise intrakorporal (mit seinem Rotor) und teilweise extrakorporal (mit seinem Stator) angeordnet.

Solch ein elektromotorbasiertes System ist einfach zu betätigen, allerdings ist auch eine Fehlbedienung verhältnismäßig leicht möglich. Somit kann eine ausreichende extrakorporale Verstellbarkeit erreicht werden, jedoch ist wegen der Gefahr eines Bedienfehlers, wobei der Elektromotor mit großer Verstellkraft eine für den Patienten gefährliche Fehlverstellung bewirkt, sachkundiges Personal zur Bedienung erforderlich. Die Zulassung eines solchen Systems ist daher aufwendig und schwierig. Die Anwendung kann meist nur durch Fachpersonal er folgen, was zusätzlichen Aufwand verursacht. Dies führt zu Abstimmungsproblemen in Bezug auf die erforderlichen Termine, an denen das Fachpersonal Zeit hat für die Behandlung (ambulanter) Patienten, oder führt zu (über)großen Verstelleinheiten (in cm Verstellweg je Termin), um die Anzahl der Besuche des Patienten zu minimieren (wenn auch um den Preis von medizinisch eventuell fragwürdig großen Verstelleinheiten je Termin).

Aus der Schrift CN 102 895 048 A ist eine extrakorporale Antriebseinheit für ein extrakorporal längenverstellbares Implantatsystem mit den Merkmalen des Oberbegriffs von Anspruch 1 vorbekannt.

Aus der Schrift US 20110230883A1 ist eine weitere extrakorporale Antriebseinheit für ein extrakorporal längenverstellbares Implantatsystem vorbekannt.

Der Erfindung liegt die Aufgabe zugrunde, ein verbessertes System zu schaffen, das eine extrakorporale Betätigung erlaubt und einfacher in der Handhabung ist.

Die erfindungsgemäße Lösung liegt in den Merkmalen der unabhängigen Ansprüche. Vorteilhafte Weiterbildungen sind Gegenstand der abhängigen Ansprüche.

Bei einer extrakorporale Antriebseinheit für ein extrakorporal längenverstellbares Implantatsystem umfassend eine Implantatkomponente mit einer von einem Antriebselement angetriebenen Verstelleinrichtung der Implantatkomponente, wobei die extrakorporale Antriebseinheit zur Betätigung des Antriebselements mittels eines Magnetfelds ausgebildet ist, wird die Aufgabe dadurch gelöst, dass die extrakorporale Antriebseinheit einen extrakorporalen Magnetring und eine Betätigungseinrichtung aufweist, wobei der extrakorporale Magnetring um eine Drehachse des Magnetrings drehbar gelagert ist, die senkrecht zu einer Ringebene des extrakorporalen Magnetrings verläuft, wobei der extrakorporale Magnetring einen Innenraum zur Aufnahme eines zu verlängernden Knochens mit der Verstelleinrichtung ausgebildet ist, wobei die Betätigungseinrichtung dazu ausgebildet ist, den extrakorporalen Magnetring mechanisch um die

Innenachse zu rotieren, wobei der extrakorporale Magnetring mittels einer Vielzahl von permanente Sub-Magneten gebildet ist, dadurch gekennzeichnet, dass die Sub-Magnete entlang des Magnetrings regelmäßig mit verschiedener Orientierung der Magnetisierung winkelfest eingesetzt sind, so dass sie in dem Innenraum ein gerichtetes statisches homogenes magnetisches Feld erzeugen, das ringfest ist.

Zuerst seien einige verwendete Begriffe erläutert:
Unter "ringfest" wird eine Anordnung verstanden, die fix in Bezug auf den Ring ist und mit dem Ring mitrotiert.

Unter "gerichtet" wird eine typischerweise einheitliche Ausrichtung eines Magnetfelds verstanden. Insbesondere soll die Ausrichtung des Magnetfelds an jeder Stelle des Feldes die gleiche Richtung aufweisen. Als "homogen" wird ein im wesentlichen gleichförmiges Magnetfeld bezeichnet, bei dem lokale Abweichungen der Feldstärke in einem mindestens 80% des Gesamtgebiets umfassenden Zentralgebiet höchstens +/- 10% vom Mittelwert betragen.

Unter "statisch" wird ein Magnetfeld von gleichbleibender Stärke verstanden. Es bildet so insbesondere einen Gegensatz zu den sich typischerweise schnell ändernden elektromagnetischen Feldern.

Die Erfindung fußt auf dem Gedanken, sich von dem elektromotorisch betätigten Konzept zu lösen und stattdessen eine mechanische Betätigung vorzusehen. Die Erfindung erreicht dies, indem sie mittels des Magnetrings ein statisches Magnetfeld bereitstellt, das mechanisch (manuell oder motorisch) gedreht werden kann. Vorzugsweise wird der Magnetring als Ganzes von der Betätigungseinrichtung rotiert. Der innerhalb der Implantatkomponente als Antriebselement fungierende Permanentmagnet richtet sich stets gemäß dem statischen Magnetfeld des Magnetrings aus. Die Stellung des als Antriebselement fungierenden Permanentmagneten innerhalb der Verstelleinrichtung ergibt sich also aus der Position bzw. der erfolgten Drehung des statischen Magnetfelds. Mit dem Übergang auf ein mechanisch gedrehtes statisches Magnetfeld erreicht die Erfindung eine bedeutende Vereinfachung und eine genaue Positionskontrolle über die Position des als Antriebselement fungierenden Permanentmagneten. So kann eine simple manuelle Betätigung genügen; Strom zur Betätigung wird nicht mehr benötigt.

Damit entfällt auch jede Gefahr eines elektrischen Schlages durch eine Fehlfunktion. Ferner entfällt auch jedes Risiko, dass es durch eine Fehlfunktion zu einem Durchgehen bzw. Weg laufen der Positionsverstellung kommen kann, was zu einer übergroßen Verstellung und damit zu einer Verletzung des Knochens selbst und insbesondere seiner umgebenden Weichteile, wie Muskeln oder Sehnen, führen kann. Außerdem wird dank des gleichbleibenden statischen Magnetfelds eine Belastung des Patienten durch Wirbelströme, wie sie erzeugt werden durch sich ändernde elektromagnetische Felder, vermieden.

Das erfindungsgemäße System mit seiner auf statischem Magnetfeld beruhenden Antriebseinheit kann somit auch von Hilfskräften bzw. Laien bedient werden, im Grunde auch von dem Patienten selbst. Dies stellt in der medizinischen Praxis einen erheblichen Vorteil dar, da somit der Patient selbst zu Hause bei Bedarf die Verstelleinrichtung betätigen kann. So ist es ermöglicht, die Verstellung häufiger und dann in schonenderen kürzeren Abschnitten vorzunehmen, als es bisher im Stand der Technik möglich war, insbesondere wenn eine Bedienung nur durch eingewiesene Fachpersonal erfolgen durfte.

Ferner erlaubt die Erfindung mit dem drehbaren Magnetring eine präzise Kontrolle über die Ausrichtung des statischen Magnetfelds. Es kann so eine Feineinstellung der Winkelposition des Antriebselements der Verstelleinrichtung erreicht werden, was eine sehr genaue Einstellung der erreichten Verstellung ermöglicht.

Die Erfindung erreicht so auf verblüffend einfache Weise eine Erhöhung der Verstellsicherheit bei gleichzeitiger Vereinfachung der Antriebseinheit, was sie somit laien- und patiententauglich macht.

Vorzugsweise ist der Magnetring teilbar in mindestens zwei Segmente, um so einen öffenbaren und verschließbaren Zugang für ein mit dem Prothesenschaft versehenes Körperteil, insbesondere Bein oder Arm, zum Innenraum des Magnetrings zu schaffen. Damit können der Magnetring und damit die Antriebseinheit als Ganzes auf einfache Weise an der Stelle angeordnet werden, an der die Implantatkomponente mit der Verstelleinrichtung im Körper angeordnet ist. Beispielsweise kann durch Aufklappen des Magnetrings die Antriebseinheit ohne weiteres im Bereich des Oberschenkels eines Patienten positioniert werden, um die in dem Schaft eines im Femur angeordnete Implantatkomponente zu verstellen. Ein Durchfädeln der zu behandelnden Extremität durch den Magnetring entfällt damit. Vorzugsweise ist der Rest der Antriebseinheit so gestaltet, dass mit dem Aufklappen des Magnetrings ein freier Zugang zu dessen Innenraum ermöglicht ist. Die Anwendung ist dank des aufklappbaren Magnetrings so mit beträchtlich erleichtert.

Zweckmäßigerweise ist ferner vorgesehen, dass das Aufklappen des Magnetrings durch die Magnetkraft der in dem Magnetring angeordneten Magnete weder verhindert noch gefördert wird. Dazu ist der Magnetring so gestaltet, dass er magnetkraftfrei aufklappbar ist. Unter "magnetkraftfrei" wird hierbei verstanden, dass die von der Magnetkraft gegen ein Aufklappen gerichtete Kraft entweder Null oder so gering ist, dass sie ein Bediener von Hand ohne weiteres, insbesondere ohne Hinzunahme von Werkzeug, überwinden kann. Mit Vorteil ist dazu eine Trennebene zwischen den Segmenten so gewählt, dass sie sich in Richtung des gerichteten statischen magnetischen Felds er streckt. Damit wird erreicht, dass quer zur Trennebene laufende Anteile der Magnetkraft minimiert sind.

Hierbei sind vorzugsweise die Segmente mit Verbindungsmitteln verbunden, von denen mindestens eines offen- und wiederverschließbar und mindestens eines scharnierartig klappbar ist.

Dank dieser Verbindungsmittel können die Segmente leicht geöffnet werden und nach Platzieren der betroffenen Extremität (das ist derjenige Körperteil, in dem die Implantatkomponente mit der Verstelleinrichtung enthalten ist, insbesondere ein Arm oder ein Bein) wieder geschlossen werden. In dem eines der Verbindungsmittel eine scharnierartige Klappbarkeit ermöglicht, kann ein vollständiges Abnehmen des öffenbaren Segments vermieden werden (obwohl auch das grundsätzlich möglich wäre). Die Handhabung wird damit weiter erleichtert. Im geöffneten, aufgeklappten Zustand ist der Magnetring vor zugsweise gegen (unerwünschte) Bewegung gesichert. Dazu ist zweckmäßigerweise eine Arretiervorrichtung vorgesehen, die den Magnetring mit seinen Segmenten in der Öffnungsposition verdrehsicher fixiert. Damit wird dem Risiko einer ungeplanten Bewegung des Magnetrings, die auch zu einem unerwünschten Zu klappen führen könnte, auf wirksame Weise begegnet. Weiter vorzugsweise ist in sozusagen umgekehrten Sinne eine Sicherungseinrichtung vorgesehen, die insbesondere mittels einer Zwangsführung dazu ausgebildet ist, eine Koppelung des Magnet rings in der geschlossen Position zu bewirken. Es wird so ein Aufklappen des Magnetrings außerhalb der Öffnungsposition blockiert. Damit wird ein unerwünschtes Öffnen des Magnetrings beispielsweise während der normalen Betätigung, sicher verhindert.

Der Magnetring ist mit Permanentmagneten ausgeführt. Zweckmäßigerweise ist er gebildet mittels einer Vielzahl von Sub-Magneten, die an dem Magnetring angeordnet sind. Diese ermöglicht die Nutzung handelsüblicher und damit kostengünstig verfügbarer typischer Permanentmagnete als Sub-Magnete. So können auch verhältnismäßig große Magnetringe effizient und mit verhältnismäßig wenig Gewicht hergestellt werden. Die einzelnen Sub-Magnete, Einzelmagnete, sind vorzugsweise gleichartig, und zwar insbesondere als magnetische Dipolkörper ausgeführt, vor zugsweise aus Seltene Erde-Metalle (sog. Seltenerdmagnete). Die Sub-Magnete sind hierbei vorzugsweise entlang des Magnetrings regelmäßig mit verschiedener Feldorientierung winkelfest eingesetzt. Durch die winkelfeste Anordnung behalten die Sub-Magnete im Magnetring ihrer Feldorientierung stets bei. Indem die einzelnen Sub-Magnete mit voneinander verschiedener Feldorientierung eingesetzt sind, ist es möglich, einen solchen Feldverlauf des statischen Magnetfelds zu schaffen, dass sich eine Feldkonzentration im Inneren des Magnetrings ergibt und außerhalb des Magnetrings eine Feldauslöschung. Ferner ist es möglich, auf diese Weise ein homogenes Magnetfeld innerhalb des Magnetrings zu erzeugen. Ideal wird dies erreicht durch möglichst viele und kleine Submagnete, jedoch kann bereits mit einer Anzahl von Submagneten von gut einem Dutzend (10 bis 20) bereits eine gute Approximation für ein nahezu homogenes Feld im Magnetring erreicht werden.

Weiter sind die Sub-Magnete zweckmäßigerweise so angeordnet, dass sich ihr Magnetfeld (genauer gesagt die Streufelder außerhalb der Sub-Magnete) entlang einer Trennebene zwischen Segmenten des Magnetrings jeweils maximal kompensiert. Somit kann das Magnetfeld quer zur Trennebene minimiert werden. Das ist ein besonders vorteilhafter Weg, um auf einfache und zu verlässige Weise zu erreichen, dass die Segmente sich magnetkraftfrei öffnen lassen, wie vorstehend bereits ausgeführt.

Mit Vorteil ist ein Gehäuse für die Antriebseinheit vorgesehen, das den Magnetring umgibt. Mit dem Gehäuse werden magnetische Felder außerhalb des Magnetrings, insbesondere Streufelder, kurzgeschlossen, so dass der das Gehäuse umgebende Außenraum weitgehend frei von magnetischen Feldern ist ("außenfeldfrei"). Dazu weist das Gehäuse eine oder mehrere Schirmungen auf und/oder ist vorzugsweise aus weichmagnetischem Material gebildet. Somit ergibt sich nach außen keine Beeinflussung durch das (im Innenraum recht starke) Magnetfeld des Magnetrings. Ein sicherer Transport wird damit deutlich erleichtert.

Zweckmäßigerweise ist die Antriebseinheit mit einer Positionierungseinrichtung versehen, die auf den Magnetring wirkt, insbesondere ein Umdrehungen des Magnetrings anzeigendes Zählwerk. Damit kann die Position des Magnetrings angezeigt werden, was dem Benutzer eine feine und zielgenaue Einstellung ermöglicht. Das gilt sowohl für die Winkelposition des Magnetrings wie auch für die Anzahl der Umdrehungen des Magnetrings. So kann dem Bediener beispielsweise mittels eines Zählwerks auf gut ablesbare und reproduzierbare Art eine Information über die Verstellstrecke der Verstelleinrichtung angezeigt werden, die mit der Antriebseinrichtung bewirkt worden ist.

Mit Vorteil kann ferner ein extrakorporaler Sperrschalter vorgesehen sein, der vorzugsweise an der Antriebseinheit angeordnet ist und auf ein an dem Prothesenschaft angeordnetes Verstellsperrorgan wirkt. Es kann so mittels des Verstellsperrorgans das Verstellelement der Implantatkomponente gegen unbeabsichtigte Verstellung gesperrt werden. Ist eine Verstellung mit der Antriebseinheit vorgesehen, wird das Verstellsperrorgan mittels des Sperrschalters gelöst, und damit das Verstellelement wieder freigegeben, um so die gewünschte Verstellung mittels der Antriebseinheit vornehmen zu können.

Die Antriebseinheit ist vorzugsweise mit einem Standfuß versehen. Er hält die Antriebseinheit in aufrechter Position und fungiert zugleich als Kippschutz, um so eine korrekte relative Positionierung von der Antriebseinheit zu der Implantatkomponente zu halten. Um eine initiale korrekte Positionierung zu erreichen, ist vorzugsweise der Standfuß ferner versehen mit einer richtungsorientierten Aufnahme für das Körperteil, insbesondere Arm oder Bein, in das die Implantatkomponente mit der Verstelleinrichtung implantiert ist.

Die Erfindung erstreckt sich auch auf ein extrakorporal längenverstellbares Implantatsystem nach Anspruch 15.

Ebenfalls hierin offenbart wird eine Implantatkomponente, bei der die Verstelleinrichtung so beschaffen ist, dass ein Antriebselement mit einem drehbeweglich angeordneten Permanentmagnet über ein Getriebe auf einen Spindeltrieb wirkt zum Umwandeln einer Drehbewegung des Permanentmagnets in eine distrahierende Längsbewegung, wobei das Antriebselement zur Betätigung mittels eines Magnetfelds einer extrakorporalen Antriebseinheit ausgebildet ist, und mit besonderem Vorteil zur Leistungsübertragung von dem Antriebselement auf den Spindel trieb ein Biegestab vorgesehen ist.

Dank des Biegestabs, der langgestreckt und zur Aufnahme bzw. Übertragung von Scherkräften ausgebildet ist, kann eine unter Last entstehende Verbiegung der Implantatkomponente ausgeglichen werden. Der Biegestab ermöglicht es, dass ein Winkelfehler zwischen dem Antriebselement mit dem Getriebe (Reduktionsgetriebe) einerseits und dem eigentlichen Spindeltrieb andererseits ausgeglichen werden kann. Ein solcher Winkelfehler entsteht insbesondere dadurch, dass die Implantatkomponente sich unter hoher Lastbeanspruchung verbiegt. Die sich relativ zueinander bewegenden Befestigungsteile, typischerweise Innen- und Außenrohr eines teleskopartigen Verstellmechanismus, werden dabei unter Last quer zu ihrer Mittelachse verbogen. Es besteht dann die Gefahr, dass das eigentliche Antriebselement mit dem Getriebe bezüglich seiner Achse nicht mehr exakt fluchtet mit der Achse des Spindeltriebs. Solche Fluchtungsfehler, die meist nur auf geringen Winkeldifferenzen beruhen, können zu einem Blockieren des Mechanismus führen. Dies kann mittels eines Biegestabs zwischen Antriebselement und Getriebe einerseits und Spindeltrieb andererseits auf verblüffend einfache Weise zuverlässig verhindert werden.

So kommt es auch unter hoher Last nicht zu einer Gefahr des Blockierens des Verstellelements. Zweckmäßigerweise weist dazu die Antriebsspindel eine einseitig offene zentrische Bohrung auf, in die der Biegestab eingesteckt ist. Damit ist der Biegestab zum einen gut geführt, zum anderen ergibt sich bei kompaktem Aufbau eine verhältnismäßig große Länge für den Biegestab. Dies ermöglicht es, auch einigermaßen große Winkelfehler von bis zu gut +/- 5 Grad mittels des Biegestabs aufzunehmen. Zur kraftschlüssigen Verbindung zwischen Biegestab einerseits und der Antriebspindel andererseits ist vorzugsweise am Grund der zentrischen Bohrung eine Kopplung zwischen dem freien Ende des Biegestabs und der Antriebspindel vorgesehen. Dazu ist weiter zweckmäßigerweise vorgesehen, dass der Biegestab eine Länge aufweist, die vor zugsweise mindestens einem Drittel, weiter vorzugsweise mindestens einer Hälfte, der Länge der Antriebsspindel entspricht, und insbesondere dazu ausgebildet ist, einen Winkelversatz von bis zu +/- 5 Grad aufzunehmen. Mit Vorteil ist der Biegestab entlang seines Hauptteils seitlich eingeschnürt ausgeführt. Dies erleichtert zum einen das gewünschte Verbiegen des Biegestabs und sichert zum anderen den erforderlichen Freigang des Biegestabs in der zentrischen Bohrung.

Für weitere Angaben zum Zusammenwirken der Implantatkomponente mit der Antriebseinrichtung wird auf vorstehende Beschreibung verwiesen.

Die Erfindung wird nachfolgend unter Bezugnahme auf die beige fügte Zeichnung anhand vorteilhafter Ausführungsformen näher erläutert. Es zeigen:
- Fig. 1: eine schematische Ansicht eines Knochens mit ein- gesetzter Implantatkomponente sowie einer daneben angeordneten Antriebseinheit;
- Fig. 2: eine schematische Darstellung eines statischen Magnetfelds innerhalb eines Magnetrings;
- Fig. 3a, b: schematische Darstellung eines Sub-Magneten sowie physische Darstellung zweier benachbart angeordneter Sub-Magnete;
- Fig. 4: eine Frontalansicht einer ersten Ausführungsform einer Antriebseinheit mit einem Magnetring gemäß der vorliegenden Erfindung;
- Fig. 5: eine Frontalansicht der ersten Ausführungsform in einem teilweise aufgeklappten Zustand;
- Fig. 6: eine perspektivische Ansicht einer zweiten Ausführungsform einer Antriebseinheit gemäß der vorliegenden Erfindung;
- Fig. 7: eine Schnittansicht der zweiten Ausführungsform mit einem Schnitt durch das Gehäuse und Frontalsicht auf den Magnetring; Fig. 8 eine perspektivische Ansicht der zweiten Ausführungsform in einem teilweise aufgeklappten Zustand;
- Fig. 9: eine perspektivische Ansicht der zweiten Ausführungsform in einem voll aufgeklappten Zustand;
- Fig. 10: eine perspektivische Ansicht der zweiten Ausführungsform mit teilweise entferntem Gehäuseoberteil;
- Fig. 11: eine Frontalansicht als Schnitt durch das Gehäuse der in Figur 10 dargestellten Ausführungsform;
- Fig. 12a, b: weitere perspektivische Ansichten der zweiten Aus führungsform mit aufgesetztem Gehäuseoberteil und mit teilgeschnittener Verriegelung;
- Fig. 13a, b: eine Frontal- sowie eine Seitenansicht einer dritten Ausführungsform mit schwenkbarem Schutzdeckel;
- Fig. 14: eine perspektivische Ansicht der dritten Ausführungsform;
- Fig. 15: Darstellungen zur Abschirmung am Beispiel der ersten Ausführungsform; Fig. 16 eine perspektivische Darstellung einer Implantatkomponente mit Verstelleinrichtung gemäß einem Ausführungsbeispiel;
- Fig. 17: eine Aufsicht auf die Implantatkomponente mit freigelegtem Innenraum;
- Fig. 18: eine teilgeschnittene Darstellung der Implantatkomponente gemäß Figur 17; und
- Fig. 19: eine Schnittansicht einer Spindel für eine Ver Stelleinrichtung der Implantatkomponente.

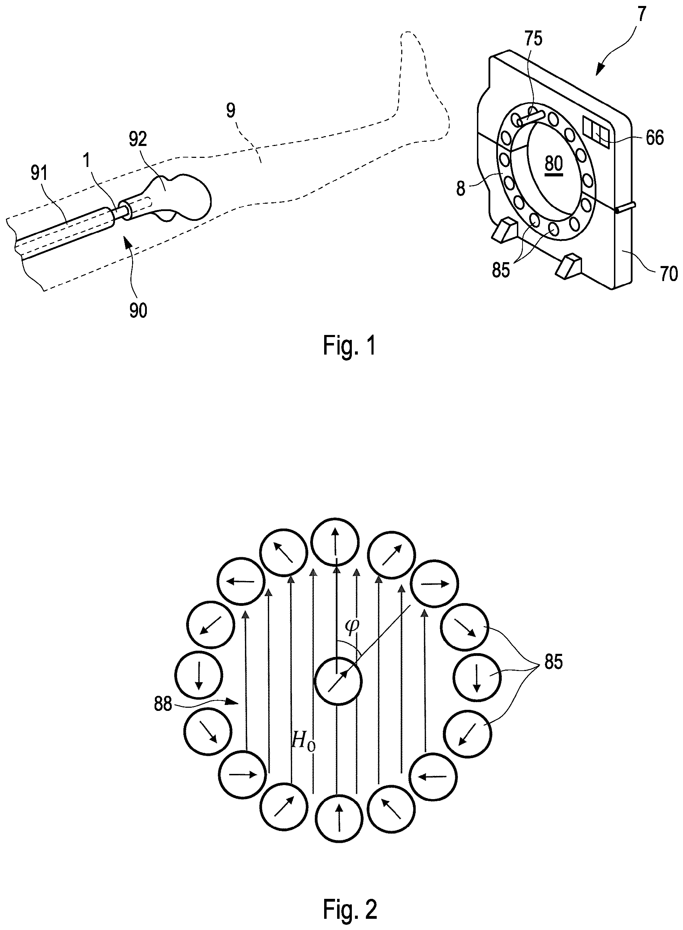

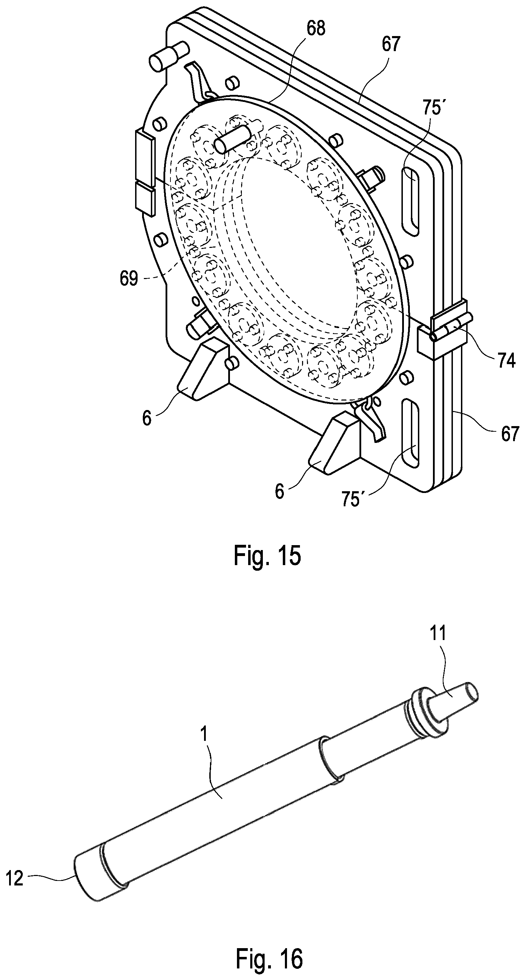

Ein System zur Längenverstellung einer implantierten Prothese sowie Verstelleinrichtung hierzu ist in Gestalt eines ersten Ausführungsbeispiels in Figur 1 schematisch dargestellt. Man erkennt eine untere Extremität, ein Bein 9, welches einen zu verlängernden Knochen 90 aufweist, in Gestalt eines Femurknochens. Er ist in zwei Teile 91, 92 geteilt, wobei in beiden Teilen die Implantatkomponente 1 verankert ist und somit die beiden Teile 91, 92 des Femurknochens 90 miteinander verbindet. Insbesondere kann es sich bei der erfindungsgemäßen Implantatkomponente 1 um einen längenverstellbaren Schaft eines Prothesensystems handeln, wie er beispielsweise in Figur 16 abgebildet ist.

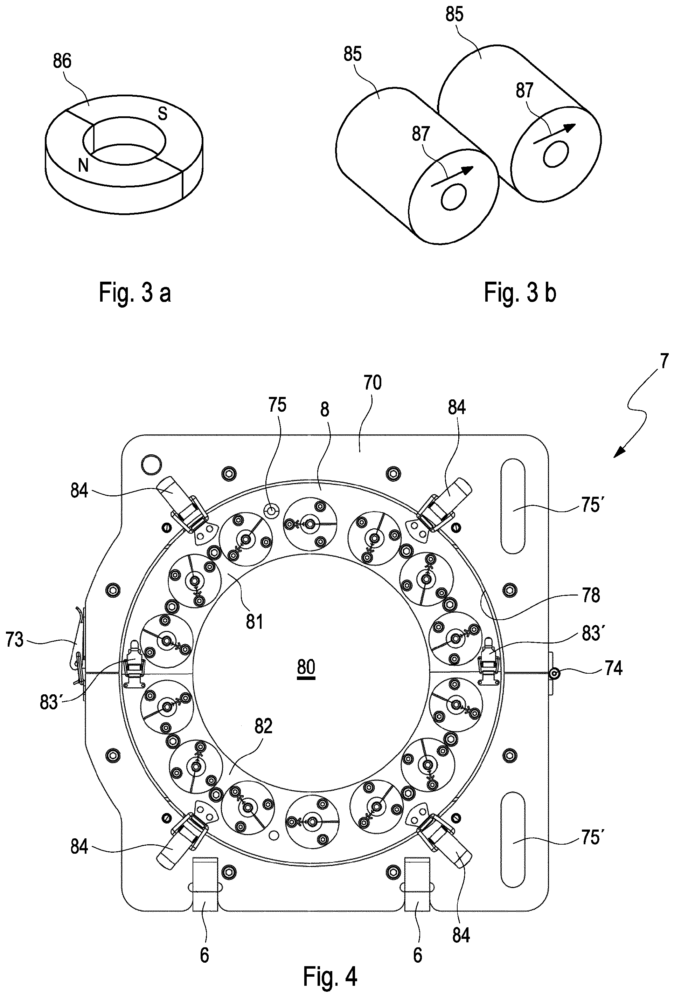

Zur Verbindung mit anderen Implantatkomponenten weist die erfindungsgemäße Implantatkomponente 1 an ihren beiden Enden jeweils einen Konusverbinder auf, vorzugsweise an einem Ende einen männlichen Konusverbinder 11 und an dem anderen Ende einen weiblichen Konusverbinder 12 (es versteht sich, dass auch andere Verbindungsformen möglich sind, bspw. mittels Gewinde etc.). Bei der Implantatkomponente kann es sich insbesondere um einen Teil einer größeren Gelenkendoprothese handeln, insbesondere für einen Totalersatz des Femurknochens 90. Mittels der Verstelleinrichtung 2 (in Figur 1 nicht dargestellt) können die Enden der Implantatkomponente 1 mit den Konusverbindern 11, 12 voneinander wegbewegt werden, d. h. die Länge der Implantatkomponente 1 kann verändert, insbesondere vergrößert werden. Zur Betätigung der Verstelleinrichtung 2 ist eine extrakorporale Antriebseinheit 7 vorgesehen. Sie umfasst einen Magnet ring 8, der drehbar innerhalb eines Trägers 70 gehaltert und geführt ist. Der Magnetring 8 ist so ausgeführt, dass in seinem Innenraum 80 ein gerichtetes statisches Magnetfeld entsteht. Ein solches gerichtetes statisches Magnetfeld 88 ist in Figur 2 dargestellt. Der Magnetring 8 ist hierbei nicht als ein unitärer Massivkörper ausgeführt, sondern ist vielmehr diskret aufgebaut, nämlich aus einer Vielzahl von Sub-Magneten 85. Die Sub-Magnete 85 sind jeweils als magnetischer Dipol ausgeführt, das bedeutet der eigentliche Magnetkörper weist auf der einen Seite einen Nordpol "N" auf der anderen Seite einen Südpol "S" auf (dies ist in Figur 3a durch unterschiedliche Grauschattierung kenntlich gemacht). Bildet man den dort dargestellten Magnetkörper weiter zu einer zylindrischen Form, so bleibt die Anordnung von Nordpol und Südpol im Grunde unverändert (dies ist in Figur 3b dargestellt, wobei die Richtung der remanenten Magnetisierung 87 durch einen schwarzen Pfeil hervorgehoben ist). Daraus ergibt sich weiterhin eine bestimmte magnetische Orientierung. Bei den Sub-Magneten 85 handelt es sich also technisch gesehen um Dipol-Magnete 86, die im Inneren ein gerichtetes magnetisches Feld aufweisen, wie in Figur 3b deutlich dargestellt ist.

Die Erfindung macht sich diese Charakteristik der Sub-Magnete 85 zu Nutze und schafft so mit einer überschaubaren Anzahl (nämlich 16) von Sub-Magneten 85 ein gerichtetes statisches Magnetfeld, das im Übrigen auch weitgehend homogen ist (siehe Figur 2). Hierbei wird unter "homogen" verstanden, dass über die Fläche des Innenraums 80 gesehen sich eine ortsabhängige Abweichung der magnetischen Feldstärke ergibt, welche nicht größer ist als +/- 15 % der tatsächlichen mittleren magnetischen Feldstärke. Erreicht wird dies durch eine bestimmte winkelmäßige Orientierung der Sub-Magnete 85 im Magnetring 8. Hierbei sind zum einen die Sub-Magnet 85 äquidistant zum Mittelpunkt angeordnet, d. h. es ergibt sich idealerweise ein gleichmäßiger Kreis. Zum anderen sind die Sub-Magnete 85 mit unterschiedlicher Orientierung in den Magnetring 8 eingesetzt.

Um dies zu visualisieren, ist in Figur 2 (in entsprechender Weise wie bei Figur 3b) die Richtung 87 der remanenten Magnetisierung eingezeichnet. Man erkennt, dass die Sub-Magnete 85 in der Mitte jeweils parallel zu dem gewünschten Verlauf der magnetischen Feldlinien stehen, aber auch nur dort. Je weiter ein Submagnet 85 zur Seite hineingesetzt wird, desto mehr wird er verdreht eingesetzt, wie sich aus der Lage des die Richtung 87 der Magnetisierung anzeigenden Pfeils ohne weiteres ableiten lässt. Es ist insbesondere diese spezielle verdrehte Anordnung der Sub-Magnete 85, die ein weitgehend homogenes gerichtetes statisches magnetisches Feld in einem Innenraum 80 hervorrufen.

Durch das Betätigen des Magnetrings 8, insbesondere mittels einer manuellen Betätigungseinrichtung 75, wird der Magnetring 8 mitsamt seinen Sub-Magneten 85 in eine Drehbewegung in der Ringebene gebracht, wodurch sich entsprechend auch das gerichtete statische Magnetfeld 88 mitdreht. Die manuelle Betätigungseinrichtung 75 kann im einfachsten Fall als Handgriff 75 oder eine griffgünstige Beschichtung der Mantelfläche des Magnetrings 8 ausgeführt sein, es kann ggf. aber auch eine Mechanisierung mittels eines Servoantriebs (nicht dargestellt) vor gesehen sein. Bezogen auf einen Permanentmagneten als Antriebselement 3 kann so durch langsames Rotieren des Magnetrings 8 mit seinem statischen Magnetfeld 88 die Winkellage des An triebs der Verstelleinrichtung 2 und der aktuell erreichte Verstellweg einfach und sicher bestimmt werden. Somit ermöglicht die erfindungsgemäße Antriebseinheit 7 schon bereits mit der manuellen Verstellung des Magnetrings 8 mittels des Betätigungselements 75 eine einfache aber dennoch präzise Steuerung und Kontrolle der tatsächlichen Position des Permanentmagneten, woraus sich wiederum die aktuelle Winkelstellung des Antriebs der Verstelleinrichtung 2 ergibt. Wegen der Stärke des in dem Hohlraum 80 des Magnetrings 8 erzeugten Magnetfelds und der Auslegung als ein statisches, d.h. gleichbleibendes starkes (wenn auch ggf. langsam rotierendes) Magnetfeld kommt dank dieser Anordnung ein die Positionierungsgenauigkeit verschlechternder Schlupf praktisch nicht vor. Es kann dem Benutzer diesbezüglich die Erfassung des erreichten Verstellwegs erleichtert werden, indem optional an der Antriebseinheit 7 ein Zählwerk 66 angeordnet ist, welches die Anzahl der Umdrehungen des Magnetrings 8 in der Antriebseinheit 7 überwacht und dem Benutzer anzeigt. Wegen der präzisen Übertragung der Drehbewegung des Magnetrings 8 auf den Permanentmagneten im Antrieb der Implantatkomponente 1 ist damit ein eindeutiges Maß für die von dem Verstelleinrichtung 2 der Implantatkomponente 1 bewirkte Verstellstrecke gegeben.

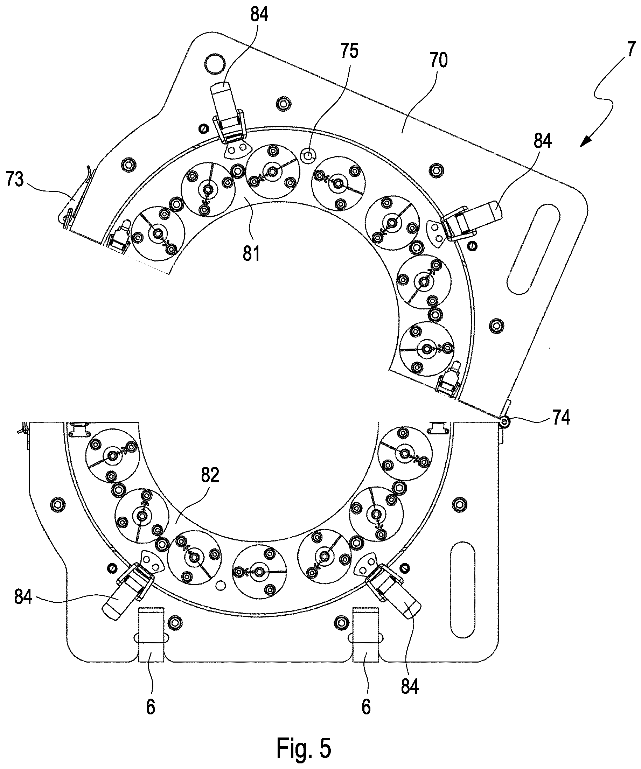

Ein Beispiel für eine konstruktive Ausführung der Antriebsein heit 7 mit Magnetring 8 wird nachfolgend unter Bezugnahme auf die Figuren 4 und 5 anhand einer ersten beispielhaften Ausführungsform erläutert. Die in ihrer Gesamtheit mit der Bezugsziffer 7 versehene Antriebseinheit umfasst einen Träger 70, in dem der Magnetring 8 drehbar gelagert ist. Hierbei fungiert die Innenseite der kreisförmigen Ausnehmung als Führung 78 für den Magnetring 8. Ferner sind im Träger 70 zwei Eingriffe 75' ausgeformt, die als Handgriffe zum Tragen vorgesehen sind. Sie ermöglichen ein problemloses Bewegen und Positionieren der Antriebseinheit 7. Im unteren Bereich der Antriebseinheit 7 sind zwei Standfüße 6 vorgesehen. Sie dienen dazu, die Antriebseinheit 7 in aufgerichteter Position kippsicher zu halten, so dass die Extremität (Bein 9) mit der Implantatkomponente 1 durch den Innenraum 80 des Magnetrings 8 geführt werden kann.

Der Magnetring 8 ist in zwei Segmente 81, 82 unterteilt, die jeweils als Halbkreisbogen ausgeführt sind. Der Magnetring 8 ist mit seinem Außenumfang über eine an einer Innenseite einer komplementär geformten kreisförmigen Ausnehmung im Träger 70 gelagert.

Der Träger 70 ist in eine obere und untere Hälfte geteilt entlang einer horizontal verlaufenden Teilungslinie. Für diese Teilungslinie sind an einer Lateralseite des Trägers 70 ein Schnallenverschluss 73 und an seiner gegenüberliegenden Late ralseite ein Scharnier 74 vorgesehen. Durch Öffnen des Schnallenverschlusses 73 kann die obere Hälfte des Trägers 70 nach oben hin aufgeklappt werden, und zwar mit dem Scharnier 74 als Drehpunkt. Beim Aufklappen verbleibt das Segment 81 des Magnetrings 8 an der oberen Hälfte und das Segment 82 des Magnetrings 8 an der unteren Hälfte des Trägers 70. Um ein Her ausfallen bzw. Verdrehen der Segmente 81, 82 zu vermeiden, sind zur Arretierung des Magnetrings 8 an dem Träger 70 insgesamt vier Arretierverschlüsse 84 vorgesehen, je zwei für eines der Segmente 81, 82. Im geschlossenen Zustand sind die beiden Segmente 81, 82 miteinander verbunden durch Spannverschlüsse 83'. Sind sie geschlossen, bilden die beiden Segmente 81 und 82 den einheitlichen Magnetring 8.

Der Magnetring 8 umfasst eine Vielzahl (bei den dargestellten Ausführungsbeispielen 14) von zylindrischen Sub-Magneten 85 aus Seltenerd-Material. Zu deren Halterung an dem Magnetring 8 sind passende Aufnahmen am Magnetring 8 vorgesehen. In diese werden die zylindrischen Sub-Magnete 85 eingesetzt und verdreh- sowie herausfallsicher befestigt. Die Verdrehsicherung ist bedeutend, da die einzelnen Sub-Magnete 85 mit genau definierter Winkel-orientierung eingesetzt und gehaltert sind. Die Winkelorientierung dient zu einer bestimmten Ausrichtung des durch den jeweiligen Sub-Magnets 85 erzeugten magnetischen Felds und ist durch Pfeile in Figur 4 und 5 visualisiert (vgl. zur Bedeutung der Pfeile auch die obige Erläuterung zu Figur 2 und 3b). Man erkennt, dass die Winkelorientierung der einzelnen Sub-Magnete 85 stets unterschiedlich von den benachbarten ist. Die Ausrichtung ist planvoll so gewählt, dass sich das Magnetfeld auf den Innenraum 80 konzentriert (der Außenraum des Magnetrings 8 ist im wesentlichen feldfrei), und so im Innenraum 80 ein im Wesentlichen homogenes, gerichtetes statisches Magnetfeld entsteht (vgl. Figur 2 und die obige Erläuterung dazu).

Durch insbesondere manuelles Verdrehen des Magnetrings 8 kann das so erzeugte statische Magnetfeld 88 rotiert werden, wobei ein sich im Innenraum 80 unter der Wirkung des Magnetfelds befindender Permanentmagnet entsprechend synchron mitdreht. Ein solcher Permanentmagnet wird als Antriebselement für die Implantatkomponente verwendet, wie später erläutert werden wird.

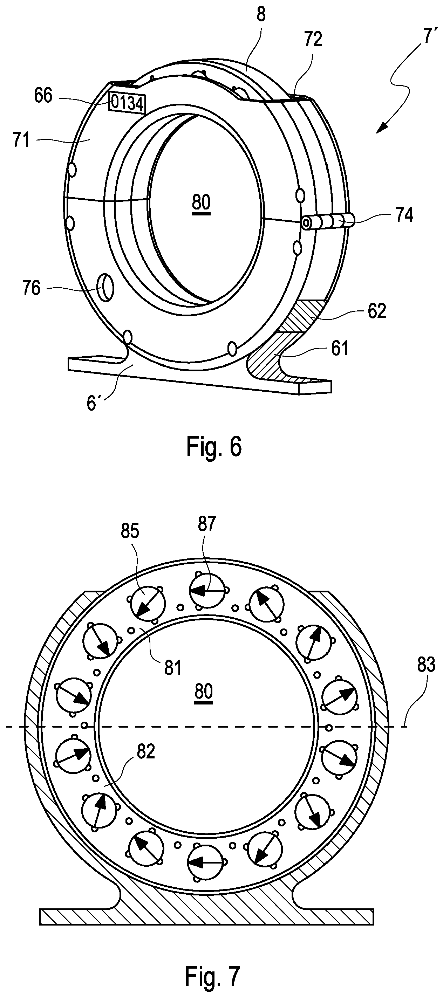

Eine zweite Ausführungsform für eine Antriebseinheit 7' ist in den Figuren 6 bis 11 dargestellt. Sie unterscheidet sich hin sichtlich ihrer Formgestaltung, ist funktional aber im Wesentlichen ähnlich zu der vorstehend beschriebenen ersten Ausführungsform. Für gleiche oder gleichartige Teile sind dieselben Bezugsziffern verwendet, wobei zur Vermeidung von unnötigen Wiederholungen auf obige Erläuterung verwiesen wird, die entsprechend sinngemäß anzuwenden ist.

Die zweite Ausführungsform der Antriebseinheit 7' weist statt des Trägers 70 ein den Magnetring umgebendes Gehäuse 71 auf.

Es umschließt den Magnetring 8 im Wesentlichen vollständig, mit Ausnahme einer im oberen Bereich des Gehäuses 71 großflächigen Öffnung 72. Sie ist so bemessen, um einen sich über mindestens 30°, mithin also ein Zwölftel des Umfangs des Magnetrings 8 erstreckenden Zugang zu ermöglichen, sodass der Benutzer den Magnetring 8 manuell verdrehen kann, insbesondere an der zur manuellen Betätigung ausgebildeten (mit Gripbeschichtung versehenen) Mantelfläche des Magnetrings 8. Es sei angemerkt, dass ein manueller Antrieb jedoch nicht zwingend ist. Für ein eventuelles Anflanschen eines Antriebsmotors ist eine Kupplungsöffnung 76 im Gehäuse 71 vorgesehen. Hier kann ein Antriebsmotor (nicht dargestellt) angeordnet werden, der auf den Magnetring 8 einwirkt und diesen in Drehung versetzt. Es sei angemerkt, dass ein solch motorischer Antrieb ggf. auch bei der ersten Ausführungsform vorgesehen sein kann.

Das Gehäuse 71 ist vorzugsweise aus weichmagnetischen Material, beispielsweise hochpermeable Eisen-, Nickel- und/oder Kobaltlegierungen, um so den Außenraum vor dem magnetischen (Rest-)Feld des Magnetrings 8 abzuschirmen. Zweckmäßig sind insbesondere niedrig legierte Bau- und Automatenstähle oder Elektrobleche .

Vorzugsweise ist zumindest das Gehäuse 71 im Bereich der Mantelfläche des Magnetrings 8 schirmend ausgeführt. Zweckmäßigerweise ist hierbei das Gehäuse 71 so bemessen, dass es im Bereich der Schirmung (z. B. Mantelfläche) einen Abstand von dem Magnetring 8 aufweist, der mindestens so groß ist wie Durchmesser/Höhe der einzelnen Sub-Magnete 85, vorzugsweise ein Mehrfaches davon. Damit kann eine gute Schirmwirkung auch mit verhältnismäßig geringen Materialstärken (typischerweise 0,5 mm oder weniger) und somit wenig Gewicht erreicht werden. Soll optional auch eine Schirmung im Bereich der Stirnseiten des Magnetrings 8 bewirkt werden, so wäre entweder das Gehäuse 71 mit recht großer Tiefe auszuführen, um den o.g. Abstand der stirnseitigen Gehäusewand von mindestens dem Durchmesser/Höhe der einzelnen Sub-Magnete 85 zu erreichen, oder die Materialstärke der stirnseitigen Gehäusewand wäre erheblich höher zu wählen als im Bereich der Mantelfläche, meist mehr als doppelt oder gar dreimal so dick (d. h. typischerweise größer als 1 mm) .

Das Gehäuse 71 ist ferner versehen mit einer richtungsorientierten Aufnahme für eine Extremität, beispielsweise einem Bein. Hier soll jedoch nicht die mit der Implantatkomponente 1 versehene Extremität aufgenommen sein, sondern deren kontralaterales Gegenstück, also das andere Bein. Das Gehäuse 71 ist dazu, wie in Figur 6 exemplarisch dargestellt, im Bereich sei nes Standfußes 6' mit einer im Querschnitt im Wesentlichen V-förmigen Ausnehmung versehen, die eine Aufnahme 61 bildet. Sie ist in dem dargestellten Ausführungsbeispiel so bemessen, dass im Bereich der Aufnahme 61 der Unterschenkel des anderen Beins (nicht dargestellt) aufgenommen werden kann oder alternativ/zusätzlich so viel größer bemessen, dass im Bereich der Aufnahme 62 der Oberschenkel des anderen Beins (nicht dargestellt) aufgenommen werden kann. Die Aufnahme 61, 62 ist zweckmäßigerweise so geformt, dass sie die Extremität nur in einer definierten Position aufnimmt. Wird dementsprechend der Unter- oder Oberschenkel des besagten anderen Beins in der Aufnahme 61, 62 platziert, so ist die Antriebseinheit 7' sozusagen automatisch korrekt ausgerichtet zur Verstellung der einzustellenden Implantatkomponente 1 (für andere Extremitäten, wie Arme, gilt entsprechendes). Dies vereinfacht für den Benutzer die korrekte Anwendung bzw. Positionierung der erfindungsgemäßen Antriebseinheit 7'. Es sei angemerkt, dass die Aufnahmen 61, 62 auch als individuell angepasste Ausformungen ausgeführt sein können.

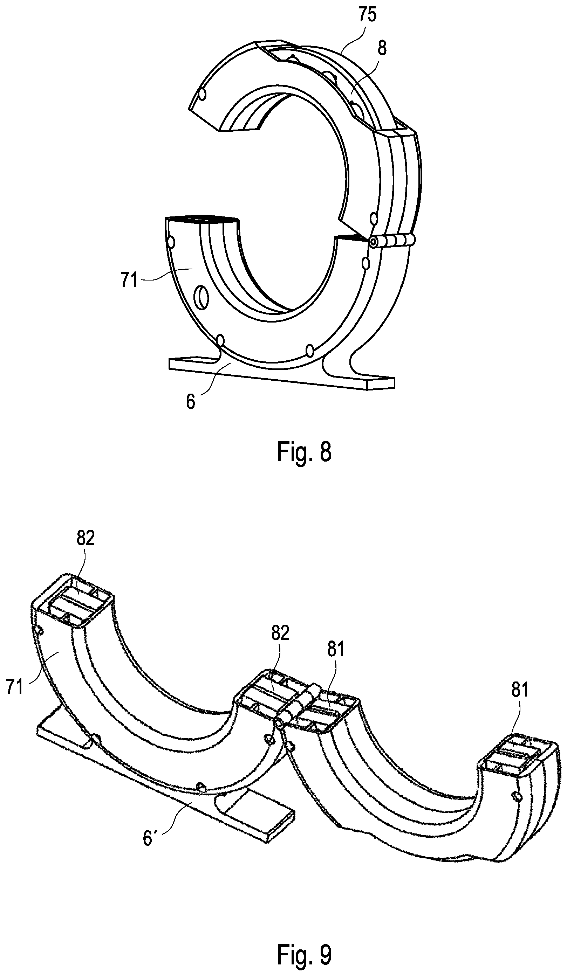

Wie auch schon bei der ersten Ausführungsform ist der Magnet ring 8 versehen mit einer Vielzahl von Sub-Magneten 85, die in der Teilschnittdarstellung in Figur 7 dargestellt sind. Man erkennt auch hier wieder die durch die Pfeile 87 visualisierte Ausrichtung der Sub-Magnete 85, um das konzentrierte gerichtete statische Magnetfeld im Innenraum 80 zu bilden. Auch hier ist wieder der Magnetring 8 in zwei Segmente 81, 82 unterteilt, welche zusammen mit der oberen Hälfte des Gehäuses 71 aufgeklappt werden können (siehe Figur 8 und 9). Wie bei der ersten Ausführungsform ist zu diesem Zweck an einer Seite ein Scharnier 74 vorgesehen. Figur 9 zeigt die zweite Ausführungsform mit vollständig aufgeklapptem Gehäuse 71. Man kann an den Trennflächen gut die jeweiligen Enden der Segmente 81, 82 erkennen, die im zusammengeklapptem Zustand (siehe Figur 7) in der Trennebene 83 liegen.

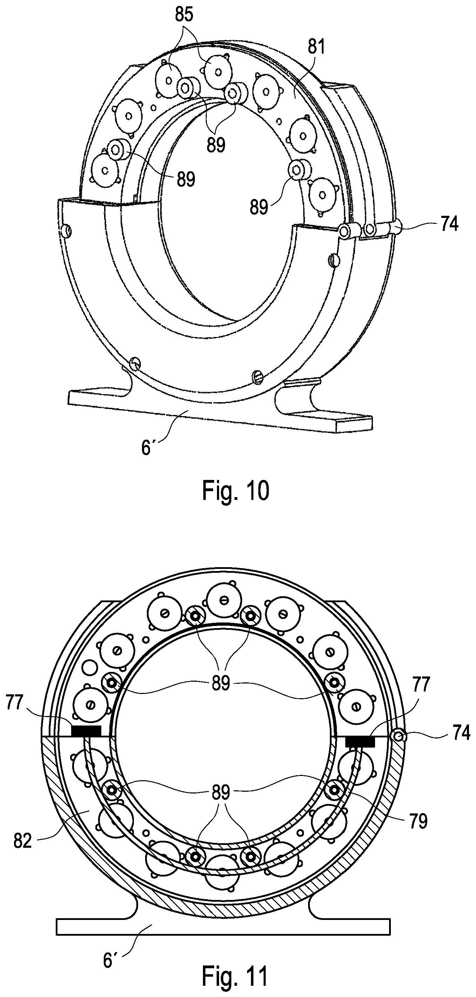

Bei der in Figur 10 gezeigten Darstellung ist ein Teil des oberen Bereichs des Gehäuses 71 entfernt. Man erkennt hierbei zusätzlich zu dem oberen Segment 81 des Magnetrings 8 deutlich dessen Submagnete 85, wie vorstehend beschrieben. Ferner er kennt man eine Mehrzahl von Führungsrollen 89 an dem Magnet ring 8, die mit einer im unteren Bereich des Gehäuses 71 angeordneten bogenförmigen Führungsschiene 79 Zusammenwirken (siehe Figur 11). Auf diese Weise wird eine sichere Zwangsführung des Magnetrings 8 in dem Gehäuse 71 erreicht. Es genügt, dass sich die bogenförmige Führungsschienen 79 über die untere Gehäusehälfte erstreckt, da sich - dank der Verbindung der Segmente 81, 82 im zusammengeklapptem Normalzustand - auf diese Weise eine vollständige Führung des Magnetrings 8 ergibt. Im aufgeklappten Zustand gilt dies zwar für das obere Segment 81 nicht, jedoch ist dies dank seiner Bogenform und eines Sperrbolzens 84' in der gleichfalls bogenförmigen oberen Hälfte des Gehäuses 71 insoweit gesichert, als dass es nicht herausfallen kann. Beim Zuklappen des Gehäuses 71 und nachfolgender gegenseitiger Verriegelung der Segmente 81, 82 wird der Magnetring 8 wieder geschlossen, und die Antriebseinrichtung 7' ist dann wieder ohne weiteres Zutun einsatzbereit.

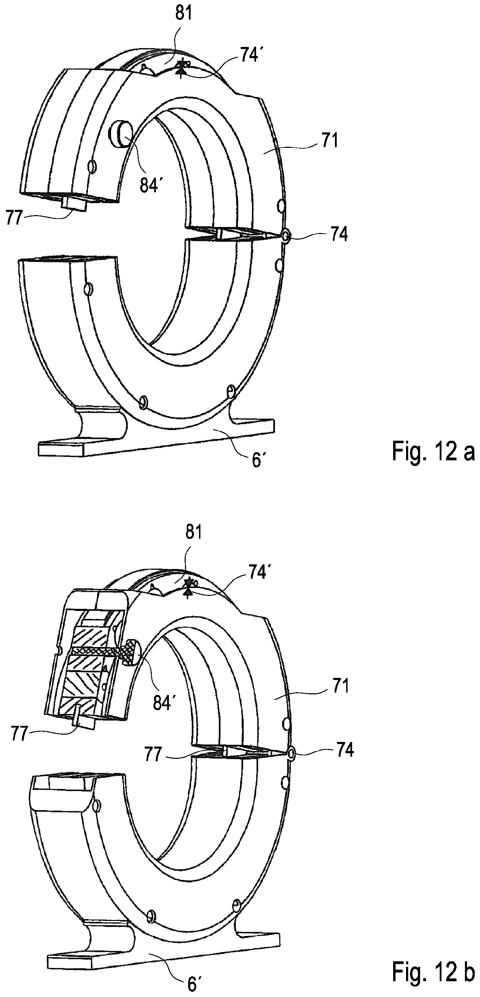

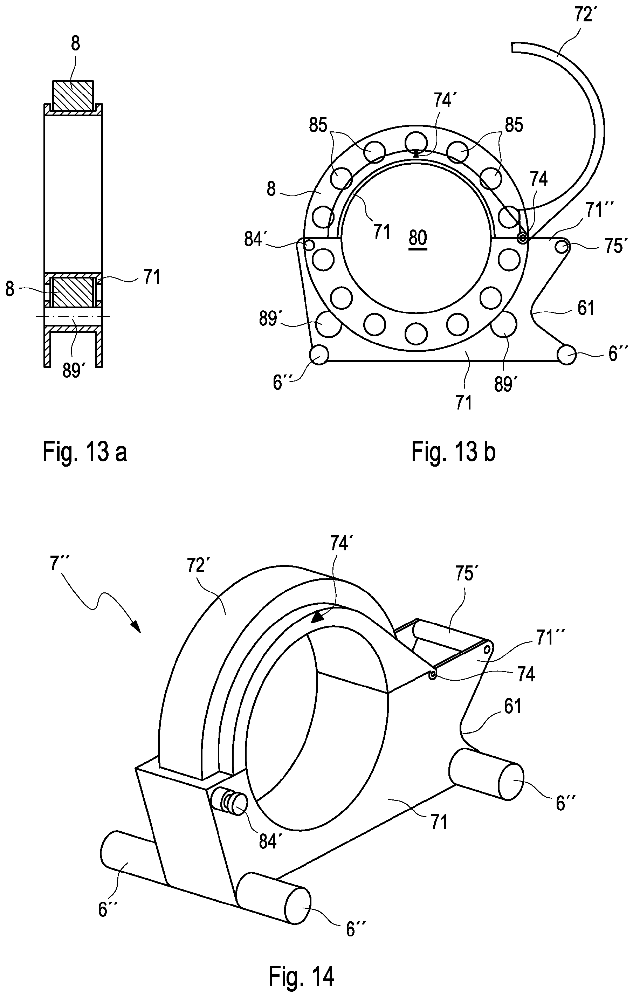

Um eine korrekte relative Positionierung der beiden Segmente 81, 82 im geschlossenen (zusammengeklappten) Zustand zu gewährleisten, sind Führungselemente 77 vorgesehen. Sie sind klinkenartig ausgeführt und an den beiden Enden der Führungsschiene 79 im Bereich der Trennebene 83 an den Segmenten 81, 82 angeordnet (s. Fig. 12 und 13). Sie sind Teil einer Federrastmechanik, welche die Segmente 81, 82 zusammen hält. Es ergibt sich so im geschlossenen Zustand eine wirksame Führung und Sicherung für den Magnetring 8, und zwar mittels der an der Führungsschiene 79 geführten Führungsrollen 89 und den Führungselementen 77 an den Enden der Führungsschiene 79.

Zur Arretierung in der gesicherten Position ist ein Sperrbolzen 84' vorgesehen, der in entsprechende Aufnahmeöffnungen im oberen Bereich des Gehäuses 71 und im oberen Segment 81 des Magnetrings 8 steckbar ist (s. Fig. 12 a, b). Die Position, in der der Magnetring 8 stehen muss zum Einstecken des Sperrbolzen 84', ist diejenige, in der die Trennebene 83 zwischen den Segmenten 81, 82 fluchtet mit dem Scharnier 74 des Gehäuses 71 - nur in dieser Grundposition ist ein Aufklappen möglich. Die korrekte Stellung des Magnetrings 8 in dieser Grundposition ist angezeigt durch eine im Bereich der Gehäuseöffnung 72 an geordnete Referenzmarkierung 74', deren eine Hälfte am Gehäuse 71 und die andere Hälfte am Magnetring 8 angebracht ist (bspw. als Doppelpfeilmarkierung) - stehen beide übereinander, ist die Grundposition erreicht.

In dieser Grundposition fluchten die Aufnahmeöffnungen in Gehäuse 71 und Segment 81 des Magnetrings 8, und der Sperrbolzen 84' kann eingesteckt werden. Damit ergibt sich eine Zwangsführung, mittels der der Magnetring 8 arretiert wird wobei zu gleich mittels mechanischer Kombination die Federrastmechanik entriegelt wird, so dass die Segmente 81, 82 entkoppelt werden und der Magnetring 8 aufgeklappt werden kann. Durch den Sperr bolzen 84' ist hierbei auch beim Aufklappen bzw. im aufgeklappten Zustand das obere Segment 81 des Magnetrings 8 gegen über unerwünschter Bewegung gesichert.

Eine dritte Ausführungsform für eine Antriebseinheit 7" ist in Fig. 13a, b und 14 dargestellt. Sie basiert auf der zweiten Ausführungsform, weist im Gegensatz zu dieser aber ein anders gestaltetes Gehäuse 71 auf. Es weist im oberen Bereich einen äußeren Schutzdeckel 72' auf, der klappbar am Scharnier 74 gelagert ist und im geöffneten Zustand den oberen Bereich des Magnetrings 8 freilegt. Im Bereich des Scharniers 74 ist ein Ausleger 71" am Gehäuse 71 vorgesehen. Er trägt zum einen den Handgriff 75' für den Transport der Antriebseinheit 7" und bildet zum anderen auf diese Weise einen Anschlag für den Schutzdeckel 72', um so eine Öffnungsbegrenzung zu erreichen.

Der Ausleger 71" ist ferner so ausgeformt, dass er mit seiner Seitenkontur eine Aufnahme 61 für die kontralaterale Extremität (das andere Bein) bietet. Im Übrigen stimmt deren Funktionalität mit derjenigen der Aufnahme 61 bei der zweiten Ausführungsform überein, so dass zur Vermeidung von Wiederholungen darauf verwiesen wird. Das gilt entsprechend auch für die anderen Komponenten der dritten Ausführungsform, die gleiche oder entsprechende Bezugsnummern wie die korrespondierenden Komponenten der zweiten Ausführungsform tragen.

Als Besonderheit weist die dritte Ausführungsform ferner ein Gegenstück zu dem äußeren Schutzdeckel 72' auf. Es ist innenseitig des Magnetrings 8 ein innerer Schutzdeckel 71' vorgesehen, der den Innenraum 80 vom Magnetring 8 separiert. Er fungiert nicht nur als mechanische Abdeckung, sondern dient auch zur Abschirmung des Umgebungsbereichs, insbesondere benachbarter Bereiche der aufzunehmenden Extremität (insbesondere Bein 9), vor unnötiger Belastung durch magnetische Felder.

Der Magnetring 8 ist gelagert auf gehäusefest angeordneten Führungsrollen 89', auf denen der Magnetring 8 in seinem unteren Bereich mit seiner Mantelfläche aufliegt. Dies ermögliche einen kompakten Aufbau mit verhältnismäßig tief liegendem Schwerpunkt. Der Gefahr eines ungewollten Umkippens der Antriebseinheit 7" kann damit begegnet werden. Optional sind die Führungsrollen 89' an ihren Stirnseiten mit vergrößertem Durchmesser ausgeführt, so dass ein Bund entsteht und eine Seitenführung für den Magnetring 8 erreicht ist. Es können auch zusätzliche Führungsrollen 89' insbesondere im oberen Be reich des Gehäuses vorgesehen sein.

Ferner weist bei dieser dritten Ausführungsform das Gehäuse 71 doppelt ausgeführte Standfüße 6" auf. Sie geben der Antriebseinheit 7" eine breitere Aufstandsfläche und erhöhen so weiter die Sicherheit gegenüber Umkippen. Im Übrigen wird zur Vermeidung von Wiederholungen auf die vorstehende Beschreibung zu der zweiten Ausführungsform verwiesen.

Am Magnetring 8 bzw. den umgebenden Gehäuse 71 und seinen Komponenten 71', 72' können magnetische Schirmungen vorgesehen sein, vorzugsweise aus magnetischen gut leitfähigem (hochpermeablen) Material, insbesondere weichmagnetische (ferromagnetische) Werkstoffe wie Eisen-, Nickel- und Kobaltlegierungen. Der Übersichtlichkeit halber sind verschiedene Schirmungen nachfolgend erläutert unter Bezugnahme auf Fig. 15 anhand der ersten Ausführungsform, wobei für die anderen Ausführungsformen entsprechendes gilt. Es kann eine Außenschirmung 67 vorgesehen sein in verhältnismäßig großen Abstand, worunter ein Vielfaches des Durchmessers bzw. der Höhe der Submagnete 85 verstanden wird. Bei einer solchen beabstandeten Schirmung sind Materialstärken des Schirmungsmaterials von nur wenigen Zehntelmillimetern ausreichend und von Vorteil.

Zur Schirmung am Magnetrad 8 ist zweckmäßigerweise eine direkt benachbarte Mantelschirmung 68 vorgesehen. Typischerweise ist hier der Abstand der Schirmung etwa gleich dem Durchmesser bzw. der Höhe der Submagnete 85, so dass Materialstärken von mindestens einem halben Millimeter, vorzugsweise bis zu einem Millimeter zweckmäßig sind. Eine solche Mantelschirmung kann auch innenseitig zweckmäßig sein, beispielsweise an dem inneren Schutzdeckel 71' der dritten Ausführungsform zum Schutz umliegender Bereiche des betroffenen Beins 9.

Weiter kann eine Schirmung auch an den Stirnflächen des Magnetrads vorgesehen sein, als Nahfeldschirmung 69. Der Abstand ist hierbei typischerweise kleiner als der Durchmesser bzw. die Höhe der Submagnete 85, so dass es zu einer starken magnetischen Aussteuerung des Weicheisens kommt. Erforderlich sind daher recht große Materialstärken von mindestens einem, meist mehreren Millimetern.

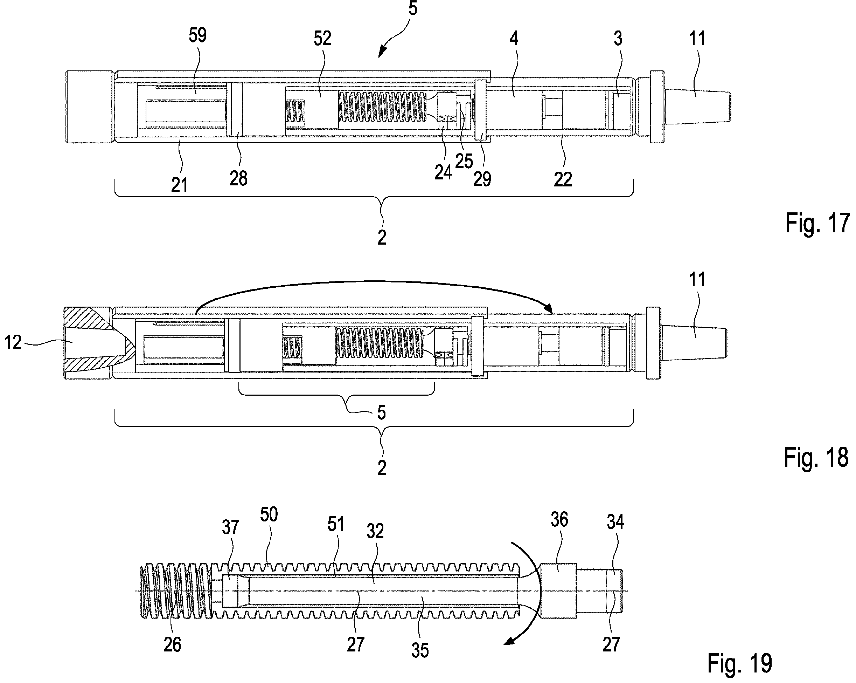

Ein Ausführungsbeispiel für eine Implantatkomponente 1 ist in den Fig. 17 bis 19 dargestellt. Figur 17 zeigt ein Teilschnitt durch die Implantatkomponente 1 mit ihrer Verstelleinrichtung 2. Die Implantatkomponente 1 ist in dem dargestellten Ausführungsbeispiel schaftartig geformt und bildet somit Teil eines Prothesenschafts, insbesondere für ein modulares Prothesensystem.

Sie weist an ihren beiden Enden jeweils ein Kopplungselement an benachbarte Prothesenmodule eines vorzugsweise standardisierten Prothesensystems auf, beispielsweise einen weiteren Schaft oder einen Anschluss an eine Gelenkkomponente. So weist die Implantatkomponente 1 an ihrem in Fig. 17 rechts dargestellten Ende einen männlichen Konus 11 als Kopplungselement und an ihrem in der Darstellung links dargestellten Ende ein als weiblichen Konus 12 ausgeführtes Kopplungselement zum Anschluss weiterer Prothesenmodule (nicht dargestellt) auf; es versteht sich, dass auch andere Verbindungsarten als Konusverbinder vorgesehen sein können.

Der eigentliche Schaftkörper zwischen den Kopplungselementen 11, 12 ist ausgeführt mit einem Außenrohr 21 und einem darin längsverschieblich geführten Innenrohr 22. Sie sind Teil einer Verstelleinrichtung 2 und wirken teleskopartig zusammen, um so die Einstellung unterschiedlicher Längen für die Implantatkomponente 1 zu ermöglichen. Das Außenrohr 21 und das Innenrohr 22 sind über zwei Gleitführungen miteinander verbunden, von denen die eine Gleitführung 28 am freien Ende des Innenrohres 22 und beweglich im Außenrohr 21 angeordnet ist, während die andere Gleitführung 29 fest am Ende des Außenrohrs 21 im Be reich des Übergangs zum Innenrohr 22 angeordnet ist. Ferner ist eine Führungsnut innenwandig am Außenrohr 21 angeordnet, um eine gegenseitige Verdrehung des Innenrohrs 22 zum Außenrohr 21 zu vermeiden. Als Lager für die Längsverstellung dient ein ebenfalls am Ende des Außenrohrs 21 angeordnetes Axiallager 25, dem ein Dichtringpaar 24 zugeordnet ist, um das Innere des Außenrohrs 21 abzuschließen gegenüber umgebenden Körpergewebe bzw. -fluiden.

Die Verstelleinrichtung 2 zur Einstellung der Länge der Implantatkomponente 1 umfasst weiter ein Antriebselement 3, welches einen drehbeweglich um eine Mittelachse des Innenrohrs angeordneten Permanentmagneten aufweist. Er ist mit einer solchen Ausrichtung angeordnet, ausgebildet, dass seine Polarisierungsrichtung (Richtung vom Nordpol zum Südpol) orthogonal zu der Mittelachse steht. Zweckmäßigerweise ist hierfür ebenfalls ein Sub-Magnet 85, wie vorstehend beschrieben, verwendet. Dem Antriebselement 3 nachgeordnet ist ein Getriebe 4 als ein Reduktionsgetriebe. In dem dargestellten Ausführungsbei spiel ist es als zweistufiges Planetengetriebe ausgeführt, dass eine Untersetzung von etwa 1 zu 20 aufweist.

Das Getriebe 4 wirkt auf einen Spindeltrieb 5, der eine Drehbewegung des Getriebes 4 umsetzt in eine Linearbewegung zur Längenverstellung. Zur Drehmomentübertragung zwischen Getriebe 4 und Spindeltrieb 5 ist ein Biegestab 35 vorgesehen. Das Ge triebe 4 wirkt auf ein getriebenes Ende 34 des Biegestabs 35, der koaxial zu der Mittelachse 27 des Innenrohrs 22 angeordnet ist. Der Biegestab 35 weist weiter einen Bund 36 auf, der im Bereich der Dichtringe 24 angeordnet ist, sowie daran anschließend einen langgestreckten eingeschnürten Schaftbereich 32 aufweist, der am freien Ende des Biegestabs 35 an einem Kopplungsstück 37 endet. Der langgestreckte eingeschnürte Schaftbereich 32 ist eingesteckt in eine einseitig offene zentrische Bohrung 51 der Antriebsspindel 50, wobei das Koppelstück 37 am Grund der zentrischen Bohrung 51 mit der Antriebsspindel 50 verbunden ist. Die von dem Biegestab 35 in Drehbewegung versetzte Antriebsspindel 50 rotiert um eine Mittelachse 26 des Außenrohrs 21 und bewirkt so eine Längsbewegung eines auf der Antriebsspindel 50 angeordneten Verstellschlittens 52, der fest mit dem Innenrohr 22 fest verbunden ist. So wird im Ergebnis durch eine Drehung des Antriebsmagneten 3 - über das Reduktionsgetriebe 4 und den Spindeltrieb 5 - eine Längsverstellung der Implantatkomponente 1 bewirkt.

Es wird nun Bezug genommen auf Figur 18 und 19. Im Betrieb kommt es unter Last zu Biegebeanspruchung in der Verstelleinrichtung 2. Eine durch die Längenverstellung der Implantatkomponente 1 entstehende wesentliche Belastung ist eine Knicklast, welche die im Wesentlichen schaftartige Implantatkomponente 1 auf Durchbiegung beansprucht. Diese Beanspruchung ist durch einen langgestreckten Pfeil in Figur 18 symbolisiert. Als Folge dieser Beanspruchung können Winkelfehler entstehen zwischen der Mittelachse 26 des Außenrohrs 21 und der Mittelachse 27 des Innenrohrs 22, d. h. unter Last fluchten die bei den Mittelachsen 26, 27 nicht mehr, sondern weisen einen Fluchtungsfehlerwinkel von einigen Grad auf.

Die empfindliche Drehmomentübertragung von dem Antriebsmagneten 3 auf den Spindeltrieb 5 kann dadurch erheblich gestört werden, wodurch es zu einem unerwünschten Blockieren kommen kann. Abhilfe bietet hier der Biegestab 35, welcher dank seiner Verbiegung den Fluchtungsfehler aufnehmen kann (symbolisiert durch die Pfeildarstellung in Fig. 19). Er ermöglicht die Kompensation von Fluchtungsfehlern im Bereich von bis zu +/- 5 Grad, vorzugsweise bis zu +/- 2 Grad. Er vergrößert somit die Toleranz des Antriebsstrangs gegenüber Biegebeanspruchung und erhöht somit die Belastbarkeit sowie Zuverlässigkeit der Verstelleinrichtung 2 der Implantatkomponente 1.

Verstellt wird die Implantatkomponente 1 mit ihrer Verstellungseinrichtung 2, indem die Extremität (Bein 9) mit der Implantatkomponente 1 so in dem Innenraum 80 der Antriebseinheit 7, 7' platziert wird, dass die Verstelleinrichtung 2 mit ihrem Antriebsmagneten 3 in der Ebene des Magnetrings 8 liegt. Durch Betätigen der Antriebseinheit 7, 7', und zwar in dem der Magnetring 8 in der Regel von Hand verdreht wird, wirkt das mit dem Magnetring 8 synchron mitdrehende Magnetfeld 88 auf den Antriebsmagneten 3, der dann ebenfalls entsprechend synchron mitgedreht wird. Durch das statische Magnetfeld, das anders als ein typisches elektromagnetisch erzeugtes Feld (insbesondere einer Elektromotorwicklung) nicht hinsichtlich seiner Stärke moduliert ist, sondern eine konstante Stärke aufweist, wird ein Schlupf zwischen Antriebsmagnet 3 und Magnetring 8 zuverlässig vermieden. Somit ist die Winkelposition wie auch die Zahl der Umdrehungen des Magnetrings 8 gleich derjenigen des Antriebsmagneten 3. Es kann so auf einfache Weise eine präzise Längenverstellung durchgeführt werden. Die Anzahl der zur Verstellung vorgenommen Umdrehungen (und damit die so erreichte Verstellstrecke) wird hierbei auf dem Zählwerk 66 für den Benutzer angezeigt.

## Patentansprüche

1. Extrakorporale Antriebseinheit (7, 7', 7") für ein extrakorporal längenverstellbares Implantatsystem umfassend eine Implantatkomponente (1) mit einer von einem Antriebselement angetriebenen Verstelleinrichtung (2) der Implantatkomponente (1), wobei die extrakorporale Antriebseinheit (7, 7', 7") zur Betätigung des Antriebselements (3) mittels eines Magnetfelds ausgebildet ist,
wobei die extrakorporale Antriebseinheit (7, 7', 7") einen extrakorporalen Magnetring (8) und eine Betätigungseinrichtung (75) aufweist, wobei der extrakorporale Magnetring (8) um eine Drehachse des Magnetrings drehbar gelagert ist, die senkrecht zu einer Ringebene des extrakorporalen Magnetrings (8) verläuft, wobei der extrakorporale Magnetring (8) einen Innenraum (80) zur Aufnahme eines zu verlängernden Knochens (90) mit der Verstelleinrichtung (2) ausgebildet ist, wobei die Betätigungseinrichtung (75) dazu ausgebildet ist, den extrakorporalen Magnetring (8) mechanisch um die Drehachse zu rotieren, wobei der extrakorporale Magnetring (8) mittels einer Vielzahl von permanente Sub-Magneten (85) gebildet ist, **dadurch gekennzeichnet, dass** die Sub-Magnete (85) entlang des Magnetrings (8) regelmäßig mit verschiedener Orientierung (87) der Magnetisierung winkelfest eingesetzt sind, so dass sie in dem Innenraum ein gerichtetes statisches homogenes magnetisches Feld erzeugen, das ringfest ist.

2. Extrakorporale Antriebseinheit (7, 7', 7") nach Anspruch 1, **dadurch gekennzeichnet, dass** das magnetische Feld auf den Innenraum (80) konzentriert ist.

3. Extrakorporale Antriebseinheit (7, 7', 7") nach Anspruch 1 oder 2, **dadurch gekennzeichnet, dass** die Sub-Magneten (85) äquidistant zu einem Mittelpunkt des Magnetrings (8) angeordnet sind.

4. Extrakorporale Antriebseinheit (7, 7', 7") nach einem der vorangehenden Ansprüche **dadurch gekennzeichnet, dass** der Magnetring (8) teilbar ist in mindestens zwei Segmente (81, 82), wobei die Segmente (81, 82) in einer Öffnungsposition des Magnetrings (8) aufklappbar sind, um so einen öffenbaren und verschließbaren Zugang für ein mit der Implantatkomponente (1) versehenes Körperteil zum Innenraum des Magnetrings (8) zu schaffen.

5. Extrakorporale Antriebseinheit (7, 7', 7") nach Anspruch 4, wobei die Segmente (81, 82) in einer Öffnungsposition des Magnetrings (8) vorzugsweise magnetkraftfrei aufklappbar sind.

6. Extrakorporale Antriebseinheit (7, 7', 7") nach Anspruch 4 oder 5, **dadurch gekennzeichnet, dass** eine Trennebene (83) zwischen den Segmenten so gewählt ist, dass sie sich parallel zu dem gerichteten statischen magnetischen Feld erstreckt.

7. Extrakorporale Antriebseinheit (7, 7', 7") nach einem der Ansprüche 4 bis 6, **dadurch gekennzeichnet, dass** die Segmente mit Verbindungsmitteln verbunden sind, von denen mindestens eines (73) öffen- und wiederverschließbar und mindestens eines scharnierartig (74) klappbar ist.

8. Extrakorporale Antriebseinheit (7, 7', 7") nach einem der Ansprüche 4 bis 7, **dadurch gekennzeichnet, dass** eine Arretiervorrichtung (84) vorgesehen ist, die den Magnetring (8) mit seinen Segmenten (81, 82) in einer Öffnungsposition verdrehsicher fixiert.

9. Extrakorporale Antriebseinheit (7, 7', 7") nach einem der Ansprüche 4 bis 8, **dadurch gekennzeichnet, dass** am Magnetring (8) eine Sicherungseinrichtung vorgesehen, die insbesondere mittels eines Sperrbolzens (84') dazu ausgebildet ist, ein Aufklappen des Magnetrings (8) außerhalb der Öffnungsposition zu blockieren.

10. Extrakorporale Antriebseinheit (7, 7', 7") nach einem der vorangehenden Ansprüche **dadurch gekennzeichnet, dass** die Sub-Magneten (85) gleichartig sind.

11. Extrakorporale Antriebseinheit (7, 7', 7") nach Anspruch 10, **dadurch gekennzeichnet, dass** die Sub-Magnete gleichartige magnetische Dipolkörper (86) sind, insbesondere gebildet aus Seltenerd-Magneten.

12. Extrakorporale Antriebseinheit (7, 7', 7") nach einem der Ansprüche 9 bis 11, **dadurch gekennzeichnet**, die Vielzahl von Sub-Magneten (85) von 10 bis 20 Sub-Magneten (85) gebildet wird.

13. Extrakorporale Antriebseinheit (7, 7', 7") nach einem der vorangehenden Ansprüche **dadurch gekennzeichnet, dass** ein Gehäuse (71) für die Antriebseinheit (7, 7', 7") vorgesehen ist, das mit einer oder mehreren Schirmungen (67, 68, 69) versehen ist und insbesondere den Magnetring (8) außenfeldfrei umgibt.

14. Extrakorporale Antriebseinheit (7, 7', 7") nach einem der vorangehenden Ansprüche **dadurch gekennzeichnet, dass** die Antriebseinheit (7, 7', 7") mit einer Positionierungseinrichtung versehen ist, die auf den Magnetring (8) wirkt.

15. Extrakorporal längenverstellbares Implantatsystem umfassend
- eine Implantatkomponente (1) für eine implantierbare Prothese, die zur Befestigung an einem zu verlängernden Knochen (90) ausgebildet ist und eine Verstelleinrichtung (2) aufweist mit zwei gegeneinander verschieblichen Befestigungsteilen (21, 22), von denen jedes an einem Teil des zu verlängernden Knochens (90) anzuordnen ist, wobei die Verstelleinrichtung (2) ausgebildet ist, die zwei Befestigungsteile (21, 22) zu distrahieren und ein Antriebselement (3) mit einem drehbeweglich angeordneten Permanentmagneten sowie ein Getriebe (4) umfasst, das in der Lage ist, eine Drehbewegung des Permanentmagnets in eine distrahierende Längsbewegung der Verstelleinrichtung (2) umzusetzen; und
- eine extrakorporale Antriebseinheit (7, 7', 7") nach einem der vorangehenden Ansprüche für das Antriebselement der Implantatkomponente (1).

## Claims

1. An extracorporeal drive unit (7, 7', 7") for an extracorporeally length-adjustable implant system comprising an implant component (1) with an adjusting device (2) of the implant component (1) driven by a drive element, wherein the extracorporeal drive unit (7, 7', 7") is configured to actuate the drive element (3) by means of a magnetic field, wherein the extracorporeal drive unit (7, 7', 7") has an extracorporeal magnetic ring (8) and an actuating device (75), wherein the extracorporeal magnetic ring (8) is rotatably mounted about an axis of rotation of the magnetic ring which extends perpendicular to a ring plane of the extracorporeal magnetic ring (8), wherein the extracorporeal magnetic ring (8) has an interior space (80) for receiving a bone (90) to be lengthened with the adjusting device (2), wherein the actuating device (75) is configured to mechanically rotate the extracorporeal magnetic ring (8) about the axis of rotation, wherein the extracorporeal magnetic ring (8) is formed by means of a plurality of permanent sub-magnets (85), **characterized in that** the sub-magnets (85) are regularly inserted along the magnetic ring (8) with different orientations (87) of the magnetization in an angularly fixed manner, so that they generate a directed static homogeneous magnetic field in the interior space which is ring-fixed.

2. The extracorporeal drive unit (7, 7', 7") according to claim 1, **characterized in that** the magnetic field is concentrated on the interior space (80).

3. The extracorporeal drive unit (7, 7', 7") according to claim 1 or 2, **characterized in that** the sub-magnets (85) are arranged equidistantly from a center point of the magnetic ring (8).

4. The extracorporeal drive unit (7, 7', 7") according to any one of the preceding claims, **characterized in that** the magnetic ring (8) is divisible into at least two segments (81, 82), wherein the segments (81, 82) can be folded open in an open position of the magnetic ring (8) in order to create an openable and closable access for a body part provided with the implant component (1) to the interior space of the magnetic ring (8).

5. The extracorporeal drive unit (7, 7 ', 7") according to claim 4, wherein the segments (81, 82) are preferably hinged without magnetic force in an open position of the magnetic ring (8).

6. The extracorporeal drive unit (7, 7', 7") according to claim 4 or 5, **characterized in that** a separating plane (83) between the segments is selected such that it extends parallel to the directed static magnetic field.

7. The extracorporeal drive unit (7, 7', 7") according to any one of claims 4 to 6, **characterized in that** the segments are connected by connecting means, at least one of which (73) can be opened and closed again and at least one of which is hinge-like (74).

8. The extracorporeal drive unit (7, 7', 7") according to any one of claims 4 to 7, **characterized in that** a locking device (84) is provided which secures the magnetic ring (8) with its segments (81, 82) in an open position in a rotationally secure manner.

9. The extracorporeal drive unit (7, 7', 7") according to any one of claims 4 to 8, **characterized in that** a securing device is provided on the magnetic ring (8), which is designed in particular by means of a locking bolt (84') to block the magnetic ring (8) from folding open outside the open position.

10. The extracorporeal drive unit (7, 7', 7") according to any one of the preceding claims, **characterized in that** the sub-magnets (85) are of the same type.

11. The extracorporeal drive unit (7, 7', 7") according to claim 10, **characterized in that** the sub-magnets are magnetic dipole bodies (86) of the same type, in particular formed from rare-earth magnets.

12. The extracorporeal drive unit (7, 7', 7") according to any one of claims 9 to 11, **characterized in that** the plurality of sub-magnets (85) is formed from 10 to 20 sub-magnets (85).

13. The extracorporeal drive unit (7, 7', 7") according to any one of the preceding claims, **characterized in that** a housing (71) for the drive unit (7, 7', 7") is provided, which is provided with one or more shields (67, 68, 69) and in particular surrounds the magnetic ring (8) without an external field.

14. The extracorporeal drive unit (7, 7', 7") according to any one of the preceding claims, **characterized in that** the drive unit (7, 7', 7") is provided with a positioning device that acts on the magnetic ring (8).

15. An extracorporeally length-adjustable implant system, comprising
- an implant component (1) for an implantable prosthesis, designed for attachment to a bone (90) to be lengthened and having an adjusting device (2) with two mutually displaceable attachment parts (21, 22), each of which is to be arranged on a part of the bone (90) to be lengthened, wherein the adjusting device (2) is designed to distract the two attachment parts (21, 22) and comprises a drive element (3) with a rotatably arranged permanent magnet and a gear (4) capable of converting a rotational movement of the permanent magnet into a distracting longitudinal movement of the adjusting device (2); and
- an extracorporeal drive unit (7, 7', 7") according to any one of the preceding claims for the drive element of the implant component (1).

## Revendications

1. Unité d'entraînement extracorporelle (7, 7', 7") pour un système d'implant réglable à longueur extracorporelle réglable, comprenant un composant d'implant (1) muni d'un dispositif de réglage (2) du composant d'implant (1) entraîné par un élément d'entraînement, dans laquelle l'unité d'entraînement extracorporelle (7, 7', 7") est configurée pour actionner l'élément d'entraînement (3) au moyen d'un champ magnétique, dans laquelle l'unité d'entraînement extracorporelle (7, 7', 7") présente un anneau magnétique extracorporel (8) et un dispositif d'actionnement (75), dans laquelle l'anneau magnétique extracorporel (8) est monté de manière rotative autour d'un axe de rotation de l'anneau magnétique, lequel s'étend perpendiculairement à un plan annulaire de l'anneau magnétique extracorporel (8), dans laquelle l'anneau magnétique extracorporel (8) est configuré de manière à présenter un espace intérieur (80) destiné à recevoir un os à allonger (90) avec le dispositif de réglage (2), dans laquelle le dispositif d'actionnement (75) est configuré pour faire tourner mécaniquement l'anneau magnétique extracorporel (8) autour de l'axe de rotation, dans laquelle l'anneau magnétique extracorporel (8) est formé au moyen d'une pluralité de sous-aimants permanents (85), **caractérisée en ce que** les sous-aimants (85) sont disposés le long de l'anneau magnétique (8) de manière régulière, avec une orientation (87) différente de la magnétisation, et fixés de façon angulairement rigide, de sorte qu'ils génèrent dans l'espace intérieur un champ magnétique statique homogène orienté, solidaire de l'anneau.

2. Unité d'entraînement extracorporelle (7, 7', 7") selon la revendication 1, **caractérisée en ce que** le champ magnétique est concentré sur l'espace intérieur (80).

3. Unité d'entraînement extracorporelle (7, 7', 7") selon la revendication 1 ou 2, **caractérisée en ce que** les sous-aimants (85) sont disposés à égale distance d'un point central de l'anneau magnétique (8).

4. Unité d'entraînement extracorporelle (7, 7', 7") selon l'une quelconque des revendications précédentes, **caractérisée en ce que** l'anneau magnétique (8) est divisible en au moins deux segments (81, 82), dans laquelle les segments (81, 82) sont rabattables dans une position d'ouverture de l'anneau magnétique (8), de manière à créer un accès ouvrable et refermable à l'espace intérieur de l'anneau magnétique (8) pour une partie du corps pourvue du composant d'implant (1).

5. Unité d'entraînement extracorporelle (7, 7', 7") selon la revendication 4, dans laquelle les segments (81, 82) sont, dans une position d'ouverture de l'anneau magnétique (8), de préférence rabattables sans force magnétique.

6. Unité d'entraînement extracorporelle (7, 7', 7") selon la revendication 4 ou 5, **caractérisée en ce qu'**un plan de séparation (83) entre les segments est choisi de telle sorte qu'il s'étend parallèlement au champ magnétique statique orienté.

7. Unité d'entraînement extracorporelle (7, 7', 7") selon l'une quelconque des revendications 4 à 6, **caractérisée en ce que** les segments sont reliés par des moyens de liaison, dont au moins un (73) est ouvrable et refermable, et dont au moins un (74) est articulé de manière à pouvoir être basculé à la manière d'une charnière.

8. Unité d'entraînement extracorporelle (7, 7', 7") selon l'une quelconque des revendications 4 à 7, **caractérisée en ce qu'**un dispositif de verrouillage (84) est prévu, lequel fixe l'anneau magnétique (8) avec ses segments (81, 82) dans une position d'ouverture de manière à empêcher toute rotation relative.

9. Unité d'entraînement extracorporelle (7, 7', 7") selon l'une quelconque des revendications 4 à 8, **caractérisée en ce qu'**un dispositif de sécurité est prévu sur l'anneau magnétique (8), lequel est en particulier configuré, au moyen d'un boulon de verrouillage (84'), pour bloquer le rabattement de l'anneau magnétique (8) en dehors de la position d'ouverture.

10. Unité d'entraînement extracorporelle (7, 7', 7") selon l'une quelconque des revendications précédentes, **caractérisée en ce que** les sous-aimants (85) sont de type identique.

11. Unité d'entraînement extracorporelle (7, 7', 7") selon la revendication 10, **caractérisée en ce que** les sous-aimants sont des corps dipolaires magnétiques de type identique (86), en particulier formés à partir d'aimants aux terres rares.

12. Unité d'entraînement extracorporelle (7, 7', 7") selon l'une quelconque des revendications 9 à 11, **caractérisée en ce que** la pluralité de sous-aimants (85) est constituée de 10 à 20 sous-aimants (85).

13. Unité d'entraînement extracorporelle (7, 7', 7") selon l'une quelconque des revendications précédentes, **caractérisée en ce qu'**un boîtier (71) pour l'unité d'entraînement (7, 7', 7") est prévu, lequel est pourvu d'un ou de plusieurs écrans de blindage (67, 68, 69) et entoure, en particulier, l'anneau magnétique (8) de manière exempte de champ extérieur.

14. Unité d'entraînement extracorporelle (7, 7', 7") selon l'une quelconque des revendications précédentes, **caractérisée en ce que** l'unité d'entraînement (7, 7', 7") est pourvue d'un dispositif de positionnement agissant sur l'anneau magnétique (8).

15. Système d'implant réglable en longueur de manière extracorporelle comprenant
- un composant d'implant (1) destiné à une prothèse implantable, configuré pour être fixé à un os à allonger (90) et présentant un dispositif de réglage (2) comportant deux parties de fixation (21, 22) coulissantes l'une par rapport à l'autre, chacune d'elles étant destinée à être disposée sur une partie de l'os à allonger (90), dans lequel le dispositif de réglage (2) est configuré pour distraire les deux parties de fixation (21, 22), et un élément d'entraînement (3) comprend un aimant permanent monté de manière rotative, ainsi qu'un mécanisme de transmission (4) apte à convertir un mouvement de rotation de l'aimant permanent en un mouvement longitudinal de distraction du dispositif de réglage (2) ; et
- une unité d'entraînement extracorporelle (7, 7', 7") selon l'une quelconque des revendications précédentes, pour l'élément d'entraînement du composant d'implant (1).
